# EUROPÄISCHE PATENTSCHRIFT

(11) **EP 3 010 440 B1**
(45) Veröffentlichungstag und Bekanntmachung des Hinweises auf die Patenterteilung: **02.10.2019**
(21) Anmeldenummer: 14736658.7
(22) Anmeldetag: 17.06.2014
(51) Int. Cl.: A61B 6/03, A61B 6/14, A61C 7/20, A61C 7/00, A61C 7/14, A61C 9/00, A61C 7/08, A61C 13/00

(54) **VORRICHTUNG UND VERFAHREN ZUM HERSTELLEN EINES ORTHODONTISCHEN APPARATS**
DEVICE AND METHOD FOR PRODUCING AN ORTHODONTIC APPARATUS
DISPOSITIF ET PROCÉDÉ DE FABRICATION D'UN APPAREIL ORTHODONTIQUE

(30) Priorität: 17.06.2013 DE 102013010186
(43) Veröffentlichungstag der Anmeldung: 27.04.2016
(73) Patentinhaber: Sonnenberg Consulting AG, 4313 Möhlin (CH)
(72) Erfinder: DJAMCHIDI, Cepand, 14532 Kleinmachnow (DE)
(74) Vertreter: Held, Stephan
(86) Internationale Anmeldenummer: PCT/EP2014/062727
(87) Internationale Veröffentlichungsnummer: WO 2014/202609

(56) Entgegenhaltungen:
- EP-A2- 1 728 485
- WO-A1-2007/129833
- WO-A1-2009/070480
- WO-A1-2009/158231
- US-A1- 2009 177 309
- US-A1- 2013 122 448

## Beschreibung

Die Erfindung betrifft eine Anordnung zum Herstellen orthodontischer Apparate und/oder orthodontischer Hilfsmittel, welche während der gesamten Dauer einer orthodontischen Behandlung benötigt werden, gemäß Patentanspruch 1. Außerdem betrifft die Erfindung eine Vorrichtung zum Herstellen orthodontischer Apparate, welche während der gesamten Dauer einer orthodontischen Behandlung benötigt werden, welche insbesondere mindestens eine 3D-Drucker-Einheit umfasst, gemäß Patentanspruch 5.

In diesem Zusammenhang wird das Dokument WO 2009/158231 A1 genannt.

Orthodontische Behandlungen betreffen die Bewegung von fehlpositionierten Zähen an die gewünschten Stellen in der Mundhöhle. Derartige Behandlungen werden zum einen aus kosmetischen Gründen durchgeführt, um beispielsweise Überbisse bzw. stark fehlgestellte Zähne zu korrigieren. Des Weiteren kann auch die Funktion des Kiefers durch eine orthodontische Behandlung verbessert werden, sofern beispielsweise eine bessere Okklusion während des Kauvorgangs erreicht werden soll.

Zur orthodontischen Behandlung werden beispielsweise umgangssprachlich feste oder festsitzende Zahnspangen verwendet. Dabei werden Brackets an den Zähnen eines Patienten fixiert, so dass ein Drahtbogen im geschlitzten Bereich jedes Brackets platziert werden kann. Dieser Drahtbogen bildet eine Bahn zur Führung der Bewegung der Zähne an eine gewünschte Stelle. Die Enden dieser Drahtbögen sind meist hinterden Mahlzähnen eines Patienten umgebogen.

Des Weiteren sind sogenannte "lose Zahnspangen" bzw. herausnehmbare Zahnspangen bekannt. Diese zeichnen sich dadurch aus, dass sie nicht fest im Kiefer des Patienten fixiert sind sondern individuell vom Benutzer entnommen werden können. Außerdem sind sogenannte Aligner bekannt. Die sogenannte Aligner-Therapie ist eine kieferorthopädische Behandlungsmethode zur weitgehend unsichtbaren Behandlung von leichteren Zahnfehlstellungen, die mit einer Mehrzahl von individuell gefertigten, dünnen, durchsichtigen Kunststoffschienen arbeitet. Mit Hilfe eines speziellen Computergrafikverfahrens wird ausgehend vom Istzustand der Zahnreihen, welcher in Kiefermodellen festgehalten wird, ein vorher bestimmtes Behandlungsziel dreidimensional dargestellt und in einzelne Behandlungsphasen unterteilt. Für jede dieser Phasen werden dann die einzelnen individuellen Schienen industriell produziert, die jeweils nur wenige Wochen lang getragen werden. In dieser Zeit werden die Zähne durch gezielte Druckausübung kontinuierlich in die vorher festgelegte Richtung bewegt.

Bei der Verwendung von bekannten Bracketsystemen tritt oftmals das Problem des fehlerhaften Beklebens auf. Dabei werden die Brackets nicht exakt an den idealen vorgesehen Positionen auf den Zähnen aufgeklebt, so dass unter Umständen nicht der gewünschte Behandlungserfolg eintritt. Demnach ist aus dem Stand der Technik die Möglichkeit des sogenannten indirekten Klebens der Brackets mit einem Übertragungstray bekannt. Auf Grundlage eines Abdrucks der Zahnreihen wird zunächst aus Gips ein Modell der Zähne hergestellt. Danach wird anhand des Zahnmodells ein Übertragungstray gefertigt, welches ein Negativ des Zahnmodelles darstellt. Dieses Zahnmodell wird zuvor entsprechend mit Brackets ausgestattet, so dass das Tray zusammen mit den darin befindlichen Brackets auf den Zähnen des Patienten aufgelegt wird, so dass die Brackets in einem Schritt und in einer richtigen Position auf den Zähnen aufgebracht werden können. Die Herstellung des Trays ist gemäß konventioneller Herstellungsmethode sehr aufwendig, da Abdrücke, Modelle etc. hergestellt werden müssen.

Sofern die orthodontische Behandlung auch die Verwendung von Alignern vorsieht müssen in diesem Zusammenhang weitere Herstellungsprozesse folgen, so dass für eine orthodontische Behandlung über einen längeren Zeitraum eine Vielzahl von Abdrücken, Herstellungsschritten und entsprechende Berechnungen vorzunehmen sind.

Die Aufgabe der vorliegenden Erfindung besteht darin, eine Vorrichtung zur Herstellung aller orthodontischer Apparate, welche während der gesamten Dauer einer orthodontischen Behandlung benötigt werden, also auch über mehrere Jahre hinweg, anzugeben. Außerdem ist es Aufgabe der vorliegenden Erfindung ein verbessertes Verfahren zur Herstellung aller orthodontischer Apparate, welche während der gesamten Dauer einer orthodontischen Behandlung benötigt werden, also auch über mehrere Jahre hinweg, anzugeben. Das Verfahren soll derart vereinfacht werden, dass es zunächst weniger Schritte bedarf, um orthodontische Apparate herzustellen. Des Weiteren soll es durch das neue Verfahren möglich sein, dass auch Zahnärzte bzw. Kieferorthopäden in der Lage sind orthodontische Apparate herzustellen, so dass die Einbindung eines Zahntechnikers und/oder eines externen Labors und/oder eines externen Produktionsstandortes entfällt.

Ferner wird eine weiterentwickelte Vorrichtung zur indirekten Klebung von orthodontischen Apparaten, insbesondere ein Tray, angegeben. Erfindungsgemäß wird diese Aufgabe im Hinblick auf die Anordnung durch den Gegenstand des Patentanspruches 1 und im Hinblick auf die Vorrichtung durch den Gegenstand des Patentanspruches 5 gelöst.

Die Erfindung beruht bezüglich der Anordnung zunächst auf dem Gedanken, eine Anordnung zum Herstellen aller orthodontischer Apparate und/oder orthodonitscher Hilfsmittel, welche während der gesamten Dauer einer orthodontischen Behandlung benötigt werden, anzugeben. Erfindungsgemäß weist diese Anordnung eine Vorrichtung zur digitalen Erfassung eines orthodontisch zu behandelnden Zahnbereichs, eine Datenverarbeitungseinheit und mindestens einen 3D-Drucker bzw. eine 3D-Drucker-Einheit auf. Die Datenverarbeitungseinheit steuert die mindestens eine 3D-Drucker-Einheit, wobei die Datenverarbeitungseinheit mit der Vorrichtung zur digita len Erfassung eines orthodontisch zu behandelnden Zahnbereichs verbunden ist oder verbindbar ist.

Es sei zunächst darauf hingewiesen, dass es sich bei dem orthodontisch zu behandelnden Zahnbereich um den gesamten Kiefer, d.h. um den Zahnbereich im Obersowie im Unterkiefer handeln kann. Des Weiteren ist es auch möglich, die vorgesehene Anordnung zur Herstellung von orthodontischen Apparaten zu verwenden, welche lediglich zur Behandlung einzelner Zähne dient.

Bei dem 3D-Drucker bzw. der 3D-Drucker-Einheit kann es sich vorzugsweise um einen Rapid-Prototyping-Drucker und/oder um einen CAD-/CAM-gestützten Drucker handeln. Des Weiteren kann die 3D-Drucker-Einheit auch als 3D-Herstellungseinheit verstanden werden, welche beispielsweise eine Fräseinheit umfasst. Im Folgenden wird die Erfindung anhand eines Rapid-Prototyping-Druckers erläutert, wobei dieser lediglich als ein Beispiel eines 3D-Druckers zu verstehen ist.

Bei der Datenverarbeitungseinheit handelt es sich um einen Rechner mit zugehöriger Software. Dieser Rechner bzw. die Datenverarbeitungseinheit ist mit dem/den Rapid-Prototyping-Drucker(n) verbunden oder verbindbar und steuert diese(n).

Bei den orthodontischen Apparaten und/oder orthodontischen Hilfsmitteln kann es sich beispielsweise um Vorrichtungen zur indirekten Klebung von orthodontischen Apparaten, wie z.B. ein Tray bzw. ein Übertragungstray handeln. Des Weiteren kann es sich um Aligner bzw. lose Zahnspangen bzw. Bissschienen handeln. Prinzipiell ist diese Anordnung zur Herstellung aller orthodontischer Apparate geeignet.

Bei der Vorrichtung zur digitalen Erfassung eines orthodontisch zu behandelnden Zahnbereichs kann es sich um einen intraoralen Scanner und/oder ein Röntgengerät und/oder um einen Computertomographen und/oder einen digitalen Volumentomographen handeln. Die digitale Erfassung eines orthodontisch zu behandelnden Zahnbereichs bzw. die dreidimensionale optische Aufnahme kann also mittels eines intraoralen Dentalkamerasystems erfolgen, welches beispielsweise mittels eines Triangolationsverfahrens eine dreidimensionale optische Aufnahme der zu behandelnden Zähne erstellt. Des Weiteren ist es möglich eine dreidimensionale Röntgenaufnahme des zu behandelnden Zahnbereichs anzufertigen. Eine derartige dreidimensionale Röntgenaufnahme des Oberkiefers bzw. des Unterkiefers bzw. des gesamten Kiefers bzw. der Kiefergelenke bzw. des Kiefergelenks kann beispielsweise mittels der Computertomographie (CT) oder der sogenannten digitalen Volumentomographie (DVT) erstellt werden.

In einer weiteren Ausführungsform der Erfindung ist es denkbar, dass ermittelte Röntgendaten und ermittelte Scandaten überlagert werden, sodass eine höhere Präzision bzgl. des diagnostischen Verfahrens erzielt werden kann.

Bei der digitalen Volumentomographie werden zur Berechnung dreidimensionaler Strukturen zweidimensionale Bilder als Datensatz erzeugt. Ein digitaler Volumentomograph besteht aus einer rotierenden Röntgenquelle und einem CCB-Sensor, die um 180° oder 360° um den fixierten Patienten rotiert werden. Mittels eines digitalen Volumentomographen können Schnittbilder in allen Raumebenen sowie dreidimensionale Ansichten berechnet werden.

Da die Vorrichtung zur digitalen Erfassung eines orthodontisch zu behandelnden Zahnbereichs mit der Datenverarbeitungseinheit verbunden ist oder verbindbar ist, werden die durch die Vorrichtung erfassten Daten an die Datenverarbeitungseinheit geschickt. Es sei darauf hingewiesen, dass dieses Verbinden der Vorrichtung zur digitalen Erfassung eines Zahnbereichs mit der Datenverarbeitungseinheit auch über sogenannte Wireless-Verbindungsmöglichkeiten wie WLAN, Bluetooth oder Infrarotschnittstelle erfolgen kann.

Die Datenverarbeitungseinheit kann des Weiteren mit einer Dateneingabevorrichtung verbunden sein oder ist mit einer derartigen Dateneingabevorrichtung verbindbar. D. h., dass der Rechner bzw. Computer beispielsweise mit einer Tastatur und/oder einer sogenannten Maus verbunden ist, so dass Personal wie z.B. ein Zahnarzt bzw. ein Kieferorthopäde oder ein Zahntechniker die Daten der Datenverarbeitungseinheit sichten kann bzw. diese ändern bzw. ergänzen kann. Bei der Dateneingabevorrichtung kann es sich des Weiteren um einen Touch-Screen handeln, welcher besonders einfach zu reinigen und zu desinfizieren ist. Eine Eingabe über weitere Endgeräte wie Mobiltelefone und/oder Smartphones und/oder sog. Tablets ist ebenso möglich. Hierbei ist es von besonderem Vorteil, auf den soeben genannten Endgeräten eine entsprechende Eingabeapplikation (App) zur Verfügung zu stellen. Die Datenverarbeitungseinheit kann beispielsweise zusammen mit einem Bildschirm, einer Tastatur sowie einer Maus eine Planungseinheit bilden, mit deren Hilfe die orthodontische Behandlung geplant werden kann. Hierzu weist die Datenverarbeitungseinheit ein entsprechendes Planungsprogramm auf, wodurch es möglich wird, anhand eines dreidimensional erzeugten Modells der zu behandelnden Zähne bzw. des orthodontisch zu behandelnden Zahnbereichs die orthodontische Behandlung zu planen. Diese Planung kann vom Bedienpersonal, also vom Zahnarzt, Kieferorthopäden bzw. von einem Zahntechniker individuell abgeändert werden. Die Planung bezieht unter anderem die einzelnen Behandlungsschritte und/oder die Auswahl der Behandlungsgeräte mit ein.

Zunächst wird ein Endergebnis bzw. die Endstellung jedes einzelnen Zahnes festgelegt. Es erfolgt eine Unterteilung der gesamten orthodontischen Behandlung, welche über mehrere Jahre hinweg andauern kann, in Einzelschritte bzw. einzelne Zwischenergebnisse. Diese einzelnen Zwischenergebnisse bzw. Behandlungsschritte werden mit entsprechenden orthodontischen Apparaten in Zusammenhang gesetzt, d.h. aus einer Vielzahl von orthodontischen Apparaten wird für den jeweiligen Behandlungsschritt der optimale orthodontische Apparat und/oder das optimale orthodonitsche Hilfsmittel herausgefiltert bzw. ausgewählt. Es ist des Weiteren möglich, während der andauernden Behandlung zusätzliche digitale Abbilder des orthodontisch behandelnden Zahnbereichs anzufertigen, sodass Korrekturen und/oder Änderungen an den orthodontischen Apparaten/Hilfsmitteln vorgenommen werden können. Ein Abspeichern der digitalen Abbilder ist von Vorteil, da der Behandlungsverlauf dokumentiert wird.

Anschließend erfolgt die Berechnung und virtuelle Konstruktion des jeweiligen orthodontischen Apparates, so dass Datensätze bezüglich des herzustellenden orthodontischen Apparates dazu verwendet werden können, um den Rapid-Prototyping-Drucker anzusteuern, so dass der jeweilige orthodontische Apparat mit Hilfe eines Rapid-Prototyping-Druckers gedruckt bzw. hergestellt werden kann.

Gemäß einem Aspekt beruht die Erfindung auf dem Gedanken, eine Vorrichtung zum Herstellen aller orthodontischer Apparate, welche während der gesamten Dauer einer orthodontischen Behandlung benötigt werden, einen 3D, vorzugsweise einen Rapid-Prototyping-Drucker anzugeben. Erfindungsgemäß weist der 3D- oder Rapid-Prototyping-Drucker mindestens eine Kammer zur Befüllung mit Brackets auf. Es ist also eine Vorratskammer vorgesehen, welche sogenannte Brackets bevorratet. Vorzugsweise sind mehrere derartige Kammern vorgesehen, so dass unterschiedliche Bracketgrößen sowie unterschiedliche Brackettypen und Brackets von verschiedenen Herstellern in mehreren voneinander unabhängigen Kammern gelagert bzw. vorhanden sind.

Zweckmäßigerweise ist es vorgesehen, dass der Rapid-Prototyping-Drucker mindestens eine Kammer zur Befüllung mit Draht umfasst. D. h. es ist eine Kammer vorgesehen, in welcher sich ein Draht befindet. Dieser Draht wird beispielsweise dazu verwendet, Drahtbögen herzustellen, welche im Zusammenhang mit festen Zahnspangen bekannt sind. Auch bzgl. derartiger Drahtbögen sind während einer Behandlungsphase mehrere unterschiedliche Exemplare notwendig, welche alle mit der beschriebenen Anordnung bzw. der erfindungsgemäßen Vorrichtung hergestellt werden können.

Des Weiteren ist es möglich, dass die Anordnung mindestens eine Kammer mit vorgefertigten Drahtbögen aufweist, welche z. B. mit Hilfe eines Greifarms entnommen werden können. Vorzugsweise erkennt die Anordnung alle zu entnehmenden Teile, wie Brackets, Drahtbögen oder andere Hilfselemente, an Hand von RFID-Codes und/oder QR-Codes. Sofern derartige Codes verwendet werden sollen, weist die Anordnung vorzugsweise ein optisches System auf, um die Codes zu erkennen.

Die Kammern mit Draht bzw. Brackets sind dafür vorgesehen, um beispielsweise ein Übertragungstray automatisch mit Brackets und/oder einem Drahtbogen versehen zu können.

Des Weiteren kann es vorgesehen sein, dass die Vorrichtung zum Herstellen aller orthodontischer Apparate ein automatisiertes Warenwirtschaftssystem umfasst, wobei dieses Warenwirtschaftssystem mit einer Überwachungsvorrichtung zur Überprüfung der Füllstände der mindestens einen mit Brackets befüllten Kammer und/oder der mindestens einen mit Draht befüllten Kammer und/oder der mindesten einen mit Hilfselementen versehenen Kammer in Verbindung steht bzw. das Warenwirtschaftssystem eine derartige Überwachungsvorrichtung aufweist. Außerdem ist es möglich, die vom Drucker verbrauchten Kunststoffmaterialien zu überwachen. In einer weiteren Ausführungsform der erfindungsgemäßen Vorrichtung kann mit Hilfe des Warenwirtschaftssystem eine Aufstellung hinsichtlich der während einer vollständigen Behandlungsdauer oder während einzelner Teilabschnitte zu verbrauchender Materialen für einen Patienten erstellt werden.

Um beispielsweise ein Übertragungstray mit Brackets und Drähten versehen zu können weist der Rapid-Prototyping-Drucker vorzugsweise eine Robotik mit mindestens einer Greifvorrichtung auf. Diese Robotik sieht die automatische Bestückung der Trays mit Brackets vor. Die Greifvorrichtung greift sich beispielsweise ein Bracket aus einer Kammer bzw. Bevorratungskammer und positioniert das Bracket anschließend an einer vorgesehenen Stelle am Übertragungstray. Zweckmäßigerweise sind mehrere Greifvorrichtung vorhanden.

Die Greifvorrichtung kann sich beispielsweise auf einer Schiene oder Rollen bewegen, um besser an die produzierten Teile zu gelangen. Außerdem ist es dadurch möglich, die produzierten Teile zur weiteren Verarbeitung an einer anderen Stelle im Gerät ablegen zu können. Gegebenenfalls können auch mehre Greifvorrichtungen vorgesehen sein. Des Weiteren ist es möglich, die produzierten Teile manuell aus der 3D-Drucker-Einheit zu entfernen und in den Arbeitsbereich des Greifarmes abzulegen.

In einer weiteren Ausführungsform der Erfindung kann der Rapid-Prototyping-Drucker mindestens eine Drahtbiegevorrichtung umfassen. Eine derartige Drahtbiegevorrichtung ist dafür vorgesehen, um den Drahtbogen einer festen Zahnspange bereits im Rapid-Prototyping-Drucker biegen zu können, so dass die Bestückung bzw. Verbindung der Brackets mit dem Drahtbogen bereits im Stadium der Herstellung des Übertragungstrays erfolgen kann. Auch die Verwendung von konfektionierten Bögen ist mit der erfindungsgemäßen Vorrichtung möglich.

Um die mittels des Rapid-Prototyping-Druckers hergestellten orthodontischen Apparate zu schützen weist der Rapid-Prototyping-Drucker vorzugsweise eine UV-Licht abweisende Abdeckung auf. Auch eventuell vorhandene Brackets welche direkt aus Kunststoff hergestellt sind oder mit einem Kunststoff beschichtet sind vorzugsweise in einer Bevorratungskammer vor Licht bzw. UV-Licht geschützt. Die entsprechende Bevorratungskammer weist diesbezüglich eine UV-Licht abweisende Abdeckung auf.

Des Weiteren kann es vorgesehen sein, dass der Rapid-Prototyping-Drucker eine Verpackungsvorrichtung aufweist. Demnach können fertiggestellte orthodontische Apparate automatisch verpackt werden. Zwar ist es vorgesehen, dass die orthodontischen Apparate in Arztpraxen hergestellt werden können, so dass die Herstellung der orthodontischen Apparate direkt vor dem Einsetzen selbiger in das Gebiss des Patienten erfolgen kann. Sofern alle orthodontischen Apparate für die gesamte Dauer einer orthodontischen Behandlung jedoch in einem Schritt bzw. zu einem Zeitpunkt hergestellt werden oder die Herstellung der orthodontischen Apparate nicht in der Arztpraxis sondern in einer Firma bzw. bei einem Zahntechniker erfolgt, ist eine Verpackung der hergestellten orthodontischen Apparate aus hygienischen Gründen von Vorteil. Außerdem ist es aufgrund der Verpackung der orthodontischen Apparate möglich, diese für die Zeitdauer zwischen der Herstellung des Apparates und der Eingliederung in andere Arbeitsschritte aufzubewahren.

In einem weiteren Aspekt der Offenbarung ist ein Verfahren zur Herstellung aller orthodontischen Apparate, welche während der gesamten Dauer einer orthodontischen Behandlung benötigt werden, vorgesehen.

Dabei werden folgende Verfahrensschritte durchgeführt: Erfassen eines orthodontisch zu behandelnden Zahnbereichs, wobei es sich wie bereits erwähnt sowohl um einzelne Zähne, als auch lediglich nur den Unter- bzw. Oberkiefer oder den gesamten Kiefer handeln kann. Das Erfassen eines orthodontisch zu behandelnden Zahnbereichs kann sowohl mit Hilfe eines anzufertigenden Abdrucks und einem anhand des Abdrucks zu erstellenden Modells des zu behandelnden Zahnbereichs als auch durch ein Erfassen mit Hilfe einer Vorrichtung zur digitalen Erfassung eines orthodontisch zu behandelnden Zahnbereiches erfolgen. Der Vorteil des erfindungsgemäßen Verfahrens liegt zunächst also darin, dass kein plastisches Zahnmodell notwendig ist. Sofern ein derartiges Zahnmodell jedoch bereits vorliegt oder eventuell zur verbesserten Veranschaulichung hergestellt wird, kann dies gemäß vorliegendem Verfahren ebenfalls verwendet werden. Es erfolgt demnach das Erfassen eines orthodontisch zu behandelnden Zahnbereichs an einem plastischen Zahnmodell.

Vorzugsweise sollte das Erfassen eines orthodontisch zu behandelnden Zahnbereichs mit Hilfe einer derartigen Vorrichtung erfolgen, da der Verzicht auf einen anzufertigenden Abdruck und den aufgrund des Abdrucks herzustellenden Modells in Wegfall kommen und des Weiteren entsprechendes Material gespart wird. Das Erfassen des Zahnbereichs kann in einer Weiterbildung der Erfindung mit Referenzpunkten oder Referenzelementen, welche am Patienten bzw. an den Zähnen befestigt werden, vorgenommen werden.

In einem weiteren Schritt des Verfahrens wird ein digitales dreidimensionales Modell eines orthodontisch zu behandelnden Zahnbereiches bereitgestellt. Sofern eine Vorrichtung zur digitalen Erfassung des zu behandelnden Zahnbereiches Verwendung findet, erfolgt das Bereitstellen eines digitalen dreidimensionalen Modells direkt nach dem Einscannen bzw. dem Anfertigen von Röntgenbildern bzw. digitalen Röntgenbildern (DVT) und der entsprechenden Berechnung des 3D-Modells. Anschließend wird eine automatische und/oder manuelle Erstellung eines Modells einer modifizierten Zahnanordnung vorgenommen. Diesbezüglich können dementsprechende Planungseinheiten mit einer Datenverarbeitungseinheit mit einem entsprechenden Planungsprogramm und/oder einer Tastatur und/oder einer Maus und/oder einem Bildschirm Verwendung finden. D.h. bei einer automatischen Erstellung des Modells einer modifizierten Zahnanordnung übernimmt die Erstellung vollständig das Planungsprogram. Bei einer manuellen Erstellung kann ein Bediener mit Hilfe der Tastatur und/oder einer Maus entsprechende modifizierte Zahnanordnungen vorgeben und/oder berechnen. Es ist des Weiteren möglich die automatische Erstellung mit der manuellen Erstellung eines Modells einer modifizierten Zahnanordnung zu koppeln, d.h. dass an dem automatisch erstellten Modell manuelle Veränderungen vorgenommen werden können.

Sofern ein Modell einer modifizierten Zahnanordnung endgültig vorliegt, erfolgt die Bereitstellung eines Datensatzes hinsichtlich der einzeln, nacheinander durchzuführenden orthodontischen Behandlungsschritte. Demnach wird ausgehend vom Istzustand der zu behandelnden Zähne bzw. des zu behandelnden Zahnbereiches ein Vergleich mit dem zu erreichenden Ergebnis bzw. der Endstellung der zu behandelnden Zähne eine Berechnung dahingehend vorgenommen, welche einzelnen Behandlungsschritte notwendig sind, um die Zähne von der Ist-Situation in die Endposition bzw. in die festgelegte modifizierte Zahnstellung bewegen zu können. Das Röntgen und/oder Scannen ist wichtig zur Vorhersage der Bewegung der Zähne. Somit können Therapiefehler und Schäden an den Zähnen bzw. am Kiefer verhindert werden.

Anschließend erfolgt eine Verknüpfung jedes einzeln nacheinander durchzuführenden orthodontischen Behandlungsschrittes mit einem orthodontischen Apparat aus einer Auswahl von mehreren orthodontischen Apparaten. Demnach wird automatisch und/oder manuell festgelegt, welcher orthodontische Apparat die jeweils einzelnen orthodontischen Behandlungsschritte durchführen kann bzw. für den jeweiligen Behandlungsschritt geeignet ist. Beispielsweise kann in diesem Zusammenhang festgelegt werden, dass der erste Schritt mit einer sogenannten losen Zahnspange erfolgt. Anschließend wird eine feste Zahnspange notwendig. Bezüglich der festen Zahnspange sind die Positionen der Brackets zu definieren. Außerdem sind diesbezüglich die Abmaße bzw. die Geometrien der einzelnen Drahtbögen der festen Zahnspange in zeitlicher Aufeinanderfolge, also einzelne Bogensequenzen, zu bestimmen. Abschließend kann es vorgesehen sein, dass sogenannte Aligner die Endkorrekturen vornehmen, so dass die Form und Abmaße der einzeln nacheinander zu verwendenden Aligner ebenfalls zu berechnen bzw. festzulegen sind. All die genannten Verknüpfungen der einzelnen Behandlungsschritte mit den orthodontischen Apparaten sowie die Maße der Apparate bzw. die Daten der jeweiligen orthodontischen Apparate können abgespeichert und den jeweiligen Patientenakten zugeordnet werden. Eine Variation hinsichtlich der aufgezeigten Behandlungsschritte ist selbstverständlich möglich. Auch ein erneutes Scannen und/oder Röntgen zur Korrekturmöglichkeit kann vorgesehen sein.

Nachdem die Verknüpfung jedes einzeln nacheinander durchzuführenden orthodontischen Behandlungsschrittes mit einem orthodontischen Apparat aus einer Auswahl von mehreren orthodontischen Apparaten erfolgt ist, ist eine automatische und/oder manuelle Erstellung eines digitalen Modells des ausgewählten orthodontischen Apparates vorzunehmen. Bei einer automatischen Erstellung des digitalen Modells erfolgt dies über ein sogenanntes Planungsprogramm. Bei einer manuellen Erstellung erfolgt diese Erstellung durch entsprechendes Bedienpersonal, wobei bei dieser manuellen Erstellung die entsprechenden Arbeiten im Zusammenhang mit der Datenverarbeitungseinheit vorzunehmen sind, also an einem Computer.

Vorzugsweise wird ein Skelettierungsprozess durchgeführt, aufgrund dessen der ausgewählte orthodontische Apparat bezüglich überschüssigen Materials auf ein Mindestmaß digital verkleinert wird. Bei einem sogenannten Skelettierungsprozess wird festgelegt, welche Auflageflächen bzw. Mindestdicken bezüglich des Materials des orthodontischen Apparates mindestens vorgesehen sein müssen um entsprechende Behandlungsschritte durchführen zu können. Mit Hilfe eines Skelettierungsprozesses wird zum einen Material gespart. Zum anderen wird für den Patienten ein angenehmes Tragegefühl bzw. ein höherer Tragekomfort und somit eine höhere Akzeptanz beim Patienten geschaffen.

Des Weiteren ist es möglich, dass eine zu der Druckvorrichtung zugehörige Robotik bzw. Greifvorrichtung, während des Druckvorgangs vorgefertigte Teile und/oder Element in die Apparatur einlegen kann. Bei den Elementen kann es sich beispielsweise um Schrauben oder Drähte handeln.

Abschließend erfolgt die Bereitstellung eines digitalen Datensatzes eines digitalen Modells des ausgewählten und optional skelettierten orthodontischen Apparates. Demnach wird das digitale Modell in einen digitalen Datensatz umgewandelt. Diese digitalen Datensätze können ebenfalls wie die bereitgestellten Datensätze hinsichtlich der einzeln, nacheinander durchzuführenden orthodontischen Behandlungsschritte in einer Patientenakte abgespeichert sein.

Ein sogenannter Rapid-Prototyping-Drucker wird aufgrund des digitalen Datensatzes gesteuert, so dass ein Drucken der Kunststoffbestandteile des orthodontischen Apparates erfolgen kann. Die Kunststoffbestandteile können beliebige Farben und Muster aufweisen.

Bei den orthodontischen Apparaten kann es sich, wie bereits erwähnt, um Vorrichtung zur indirekten Klebung von orthodontischen Apparaten, insbesondere Trays und/oder Aligner und/oder herausnehmbare Zahnspangen handeln. Nach dem Drucken der Kunststoffbestandteile des orthodontischen Apparates erfolgt eine automatische und/oder manuelle Bestückung des gedruckten orthodontischen Apparates mit mindestens einem Bracket und/oder mindestens einem gebogenen Draht und/oder Schrauben und/oder anderen Elementen. Diese Bestückung eines orthodontischen Apparates mit Brackets und/oder einem Draht ist beispielsweise bei der Herstellung eines Übertragungstrays notwendig. Sofern eine automatische Bestückung gewählt wird, weist der Rapid-Prototyping-Drucker eine entsprechende Robotik sowie entsprechende Bevorratungskammern bezüglich der Brackets und bezüglich des Drahtes auf. Optional kann der Rapid-Prototyping-Drucker eine Drahtbiegevorrichtung aufweisen, welche die Drahtbögen herstellen kann.

Vorzugsweise erfolgt eine automatische Mengenerfassung von in den Kammern eines Rapid-Prototyping-Druckers befindlichen Brackets und/oder Drahtes und/oder des verbrauchten und/oder benötigten Kunststoffs und/oder der in Bevorratungskammern befindlichen Schrauben oder anderen Elementen. Die erfassten Daten bezüglich der vorhandenen Mengen von Brackets und/oder vorhandenen Drahtes und/oder des Kunststoffs werden an ein Warenwirtschaftssystem weitergeleitet. Vorzugsweise ist es möglich mit Hilfe des Warenwirtschaftssystems automatische Bestellvorgänge hinsichtlich benötigter Brackets und/oder benötigten Drahts und/oder benötigten Kunststoffes und/oder benötigter Hilfselemente, wie z. B. Schrauben, zu erzeugen.

Gemäß einem weiteren Aspekt beruht die Offenbarung auf dem Gedanken, eine Vorrichtung zum indirekten Kleben von orthodontischen Apparaten, insbesondere ein Tray anzugeben, wobei dieses Tray bzw. die Vorrichtung zur indirekten Klebung mindestens eine Sollbruchstelle aufweist. Bei der Kunstruktion des Trays bzw. bei den Abmaßen des Trays fließt erstmals nicht der Gingiaverlauf in die Konstruktion mit ein.

Nach Durchführen der indirekten Klebung von beispielsweise Brackets, so dass die zunächst im Tray befindlichen Brackets und/oder der befindliche Draht auf den orthodontisch zu behandelnden Zahnbereich aufgeklebt sind, bestehen oftmals Probleme das Übertragungstray von den bereits festgeklebten bzw. fixierten Brackets und/oder dem festgeklebten bzw. befestigten Draht zu entfernen. Demnach weist das Tray vorzugsweise mindestens eine Sollbruchstelle bei dem gleichzeitigen Setzen/Arretieren mehrerer Brackets auf, so dass das Entfernen des Übertragungstrays erleichtert wird. Des Weiteren ist es möglich mit Hilfe von Hinterschnitten das Tray derart zu gestalten, dass die Entfernung des Trays zusätzlich erleichtert werden kann.

In einem weiteren Beispiel der Offenbarung handelt es sich bei dem Tray um einen skelettierten orthodontischen Apparat, welcher bezüglich überschüssigen Materials auf ein Mindestmaß verkleinert ist.

Die Erfindung wird im Folgenden anhand eines Ausführungsbeispiels unter Bezugnahme auf die beigefügten Zeichnungen näher erläutert. Darin zeigen:
- Fig. 1:: eine Anordnung zum Herstellen aller orthodontischer Apparate, welche während der gesamten Dauer einer orthodontischen Behandlung benötigt werden;
- Fig. 2:: eine erfindungsgemäße Vorrichtung zum Herstellen von orthodontischen Apparaten, welche einen 3D-Drucker, nämlich einer Rapid-Prototyping-Drucker, umfasst;
- Fig. 3:: ein Ablaufschema bezüglich eines erfindungsgemäßen Verfahrens zur Herstellung aller orthodontischer Apparate;
- Fig. 4:: ein erweitertes Ablaufschema bezüglich des erfindungsgemäßen Verfahrens zur Herstellung aller orthodontischer Apparate;
- Fig. 5:: ein nochmals erweitertes Ablaufschema des erfindungsgemäßen Verfahrens zur Herstellung von orthodontischen Apparaten; und
- Fig. 6:: eine Vorrichtung zur indirekten Klebung von festsitzenden Zahnspangen.

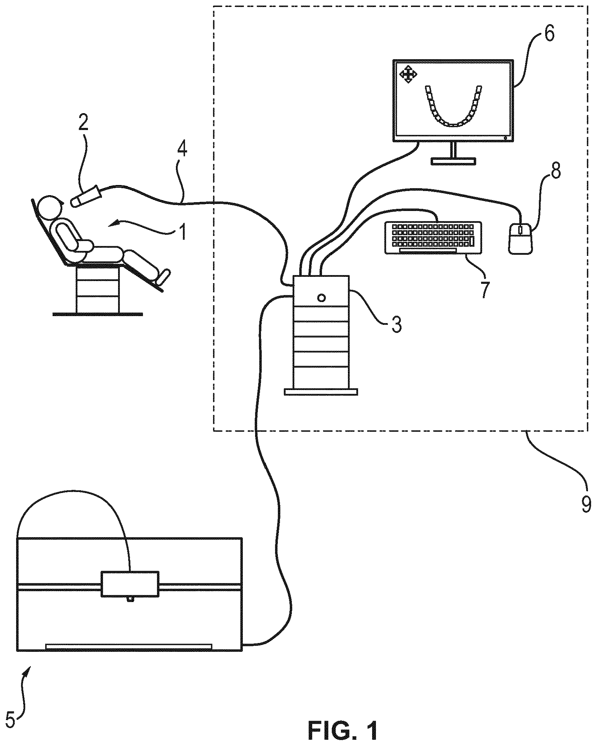

Wie in Fig. 1 dargestellt, kann sich die Anordnung zum Herstellen aller orthodontischer Apparate, welche während der gesamten Dauer einer orthodontischen Behandlung benötigt werden, in den Räumen einer Zahnarztpraxis bzw. eines Kieferorthopäden oder eines Zahnlabors befinden.

Demnach befindet sich ein Patient 1 auf einem Behandlungsstuhl, während die orthodontisch zu behandelnden Zahnbereiche oder der gesamte Kiefer mit Hilfe einer Vorrichtung zur digitalen Erfassung der Bereiche bzw. des Kiefers erfasst werden. Im vorliegenden Ausführungsbeispiel handelt es sich bei der Vorrichtung zur digitalen Erfassung um einen intraoralen Scanner 2. Dieser intraorale Scanner ist mit einer Datenverarbeitungseinheit 3 verbunden bzw. verbindbar. Im vorliegenden Fall ist eine Datenleitung 4 vorgesehen, wobei es auch möglich ist, die Verbindung mittels Bluetooth oder Infrarot oder mit Hilfe einer WLAN-Verbindung herzustellen.

Die Anordnung umfasst des Weiteren einen Rapid-Protoyping-Drucker 5, wobei dieser Rapid-Prototyping-Drucker 5 von der Datenverarbeitungseinheit 3 gesteuert wird. Es ist möglich mehrere Drucker miteinander zu koppeln. Die Datenverarbeitungseinheit 3 bildet im vorliegenden Fall zusammen mit einem entsprechenden Planungsprogramm, einem Bildschirm 6, einer Tastatur 7 sowie mit einer Maus 8 eine Planungseinheit 9.

Gemäß Ausführungsbeispiel in Fig. 1 wird eine Vorrichtung zur indirekten Klebung von orthodontischen Apparaten, nämlich ein Tray gefertigt. Allgemein ist es auch denkbar, dass lediglich das Einzelkäppchen bezüglich einzelner Zähne mit Hilfe der erfindungsgemäßen Anordnung hergestellt wird. Dies betrifft alle Ausführungsformen sowie alle in den folgenden Figuren dargestellten Ausführungsformen der Erfindung.

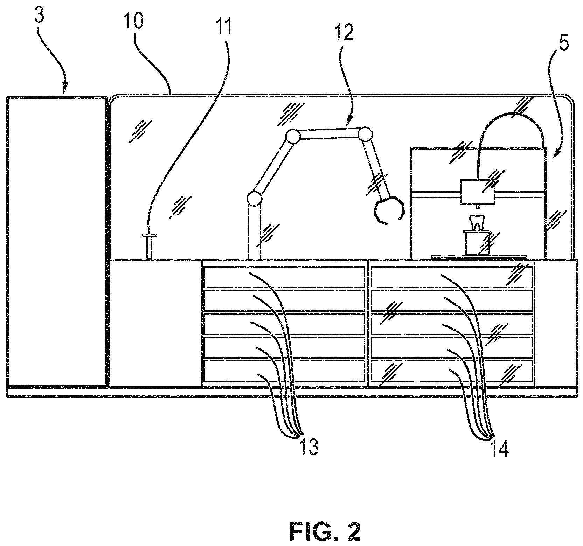

Die erfindungsgemäße Vorrichtung zum Herstellen aller orthodontischer Apparate, welche einen 3D-Drucker, insbesondere einen Rapid-Prototyping-Drucker, umfasst, wird in Fig. 2 detailliert dargestellt.

Die dargestellte Vorrichtung ist als eine Einheit zu betrachten, welche mindestens einen Rapid-Prototyping-Drucker 5 umfasst. Außerdem ist eine Datenverarbeitungseinheit 3 vorgesehen, welche im Ausführungsbeispiel gemäß Fig. 2 in baulichem Zusammenhang mit den weiteren Baugruppen steht. Ausgehend von der Datenverarbeitungseinheit 3 werden die Daten bezüglich des herzustellenden orthodontischen Apparates an den 3D-Drucker 5 gesendet. Beispielsweise mittels Rapid-Prototyping-Verfahrens werden die Kunststoffbestandteile des orthodontischen Apparates hergestellt.

Anschließend werden die hergestellten Kunststoffbestandteile beispielsweise mit Hilfe eines Greifarms 12 von der 3D-Drucker-Einheit zu einem Halter 11 transportiert. In diesem Halter 11 wird der Kunststoffbestandteil des orthodontischen Apparates beispielsweise mit weiteren Elementen und/oder Hilfsmitteln versehen. Hierbei kann es sich beispielsweise um Brackets und/oder Drahtbögen und/oder andere Hilfsmittel handeln. Die zu bestückenden Brackets und/oder Drahtbögen und/oder andere Hilfsmittel sind in Vorratsbehältern bzw. Kammern 13 bevorratet. Es kann vorgesehen sein, mehrere Brackettypen und/oder Bracketgrößen zu bevorraten. Außerdem kann es vorgesehen sein, mehrere Drähte mit unterschiedlichen Drahtdurchmessern zu bevorraten.

Mit Hilfe des Greifarms 12 werden die Brackets und/oder Drahtbögen und/oder Hilfsmittel auf dem Kunststoffbestandteil des orthodontischen Apparates platziert und optional befestigt.

Nach Fertigstellung des orthodontischen Apparates kann dieser vom Halter 11 mit Hilfe des Greifarms 12 in eine der Aufbewahrungskammern 14 transportiert werden. Optional kann die dargestellte Vorrichtung eine Verpackungseinheit aufweisen, welche die fertiggestellten Apparate beispielsweise in Kunststofffolien und/oder Behälter verpackt.

Die Vorrichtung ist außerdem mit einer Abdeckung 10 versehen. Dabei handelt es sich vorzugsweise um eine orange-gefärbte Scheibe, welche die orthodontischen Apparate vor einfallendem Licht, insbesondere UV-Licht, schützt. Die Aufbewahrungskammern 14 sind vorzugsweise ebenfalls mit einer derartigen Scheibe versehen. Dabei kann es sich auch um eine zusätzlich angebrachte UV-Scheibe handeln.

Es sei darauf hingewiesen, dass alle in Fig. 2 dargestellten Bauteile baulich miteinander verbunden sein können. Des Weiteren ist es denkbar, dass lediglich einzelne Bauteile, wie z. B. die Rapid-Prototyping-Druckeinheit 5, der Halter 11 und der Greifarm 12 fest miteinander verbunden sein können. Außerdem ist es denkbar, dass die einzelnen Bauteile lediglich nebeneinander angeordnet sein können und variabel verschiebbar sind.

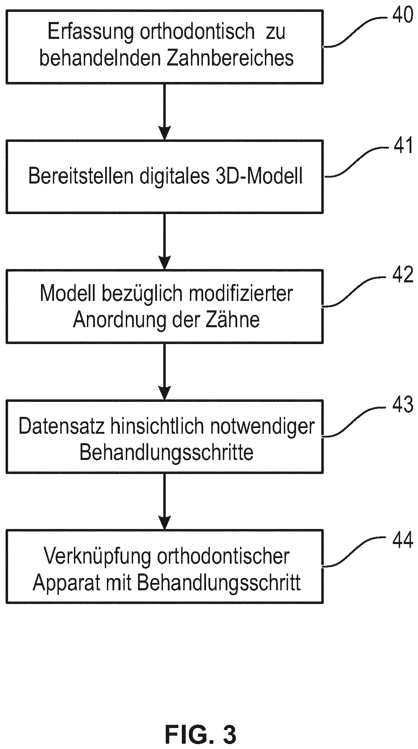

In Fig. 3 wird ein Schema bezüglich des erfindungsgemäßen Verfahrens zur Herstellung orthodontischer Apparate aufgezeigt.

Gemäß erstem Verfahrensschritt 40 erfolgt das Erfassen eines orthodontisch zu behandelnden Zahnbereichs. Beispielsweise wird mittels eines intraoralen Scanners direkt am Patienten, an einem Modell oder alternativ aus einem Abdruck ein digitales dreidimensionales Modell eines orthodontisch zu behandelnden Zahnbereiches bereitgestellt. Dieser Schritt wird mit Bezugszeichen 41 gekennzeichnet. Die Erstellung des Datensatzes kann des Weiteren auf der Grundlage von 3D/DVT-Bildern und einem Scannerbild zusammen erstellt werden, damit die Bewegung der Zahnwurzeln sichtbar gemacht wird. Somit kann kontrolliert werden, ob die geplanten Bewegungen möglich sind und die Zähne bei der orthodontischen Behandlung nicht beschädigt bzw. geschädigt werden.

Anschließend wird automatisch und/oder manuell ein Modell einer modifizierten Zahnanordnung erstellt. D.h. es wird ein virtuelles vorzugsweise dreidimensionales Modell bezüglich der endgültigen Stellung und Position jedes zu behandelnden Zahnes erstellt. Dieses Erstellen 42 kann sowohl automatisch mit einem entsprechenden Bearbeitungsprogramm als auch manuell erfolgen. Das automatische Erstellen erfolgt beispielsweise mit Hilfe eines Planungsprogrammes, wobei die Software bzw. das Programm Vorschläge bezüglich der Endposition bzw. Endstellung der Zähne vorgeben kann. Es soll jedoch auch eine manuelle Bearbeitung der automatisch vorgeschlagenen und erstellten modifizierten Zahnanordnung möglich sein. In diesem Zusammenhang kann der Zahnarzt bzw. Kieferorthopäde mit Hilfe der Tastatur und/oder der Maus und/oder einem Touchscreen und/oder einem Tablet-PC im Planungsprogramm entsprechende Änderungen an dem automatisch generierten Vorschlag vornehmen.

In einem weiteren Schritt 43 erfolgt die Bereitstellung eines Datensatzes hinsichtlich der einzeln, nacheinander durchzuführenden orthodontischen Behandlungsschritte. Demnach wird ein Vergleich des Ist-Zustandes mit dem Modell einer modifizierten Zahnanordnung vorgenommen. Die Planungssoftware bzw. der Benutzer kann die durchzuführenden Positions- und Stellungsveränderungen der Zähne in einzelne, nacheinander durchzuführende Behandlungsschritte unterteilen.

Anschließend kann jeder einzelne, nacheinander durchzuführende Behandlungsschritt mit einem orthodontischen Apparat aus einer Auswahl von mehreren orthodontischen Apparaten verknüpft werden. Dieser Schritt 44 kann wiederum automatisch und/oder manuell erfolgen, d.h. unter Zuhilfenahme eines Planungsprogrammes kann der Bediener bspw. entsprechende Änderungen an automatischen Vorschlägen vornehmen. Bei möglicherweise kritischen geplanten Bewegungen kann ein automatischer Hinweis erfolgen, sodass die Planung der orthodontischen Behandlung überdacht und korrigiert werden kann.

Im Schritt 44 können mehrere unterschiedliche orthodontische Apparate für die unterschiedlichen Behandlungsschritte als geeignet erachtet werden. Die Auswahl der jeweiligen orthodontischen Apparate kann beispielsweise in einer Patientenakte hinterlegt bzw. gespeichert werden.

Nach Festlegung der einzeln, nacheinander zu verwendenden orthodontischen Apparate 44 kann eine automatische und/oder manuelle Erstellung eines digitalen Modells des ausgewählten orthodontischen Apparates erfolgen. Die Erstellung eines digitalen Modells kann zu diesem Zeitpunkt für alle ausgewählten orthodontischen Apparate erfolgen. Des Weiteren ist es denkbar, dass die Erstellung eines digitalen Modells nach Abschluss und vor Beginn jedes einzelnen Behandlungsschrittes erfolgt, sofern die dafür notwendigen Datensätze abgespeichert bzw. in einer Patientenakte abgespeichert werden. Sofern zu irgendeinem Zeitpunkt während der gesamten Dauer einer orthodontischen Behandlung unvorhergesehene Entwicklungen eintreten, d.h. dass Zähne nicht wie vorhergesehen ihre Position verändern, ist es möglich, dass während der gesamten orthodontischen Behandlung die entsprechend zu behandelnden Zahnbereiche nochmals erfasst werden (Schritt 40) sowie nochmals ein digitales 3D-Modell bezüglich der erfassten Zahnbereiche erstellt wird (Schritt 41). Es ist des Weiteren möglich, während der andauernden Behandlung zusätzliche digitale Abbilder des orthodontisch behandelnden Zahnbereichs anzufertigen, sodass Korrekturen und/oder Änderungen an den orthodontischen Apparaten/Hilfsmitteln vorgenommen werden können. Ein Abspeichern der digitalen Abbilder ist von Vorteil, da der Behandlungsverlauf dokumentiert wird.

Optional kann nach der Erstellung eines digitalen Modells des ausgewählten orthodontischen Apparates ein Skelettierungsprozess 51 durchgeführt werden. Mit Hilfe eines Skelettierungsprozesses kann das digitale Modell des ausgewählten orthodontischen Apparates bezüglich überschüssigen Materials auf ein Mindestmaß digital verkleinert werden. Dies führt zu einem geringeren Materialverbrauch. Des Weiteren wird das Tragegefühl bzw. der Tragekomfort und somit die Akzeptanz beim Patienten verbessert.

Es folgt die Bereitstellung eines digitalen Datensatzes 52 des digitalen Modells des ausgewählten und optional skelettierten orthodontischen Apparates.

Die Steuerung eines Rapid-Prototyping-Druckers erfolgt anschließend aufgrund des zur Verfügung gestellten digitalen Datensatzes, so dass die Kunststoffbestandteile des orthodontischen Apparates gedruckt werden können (Schritt 53).

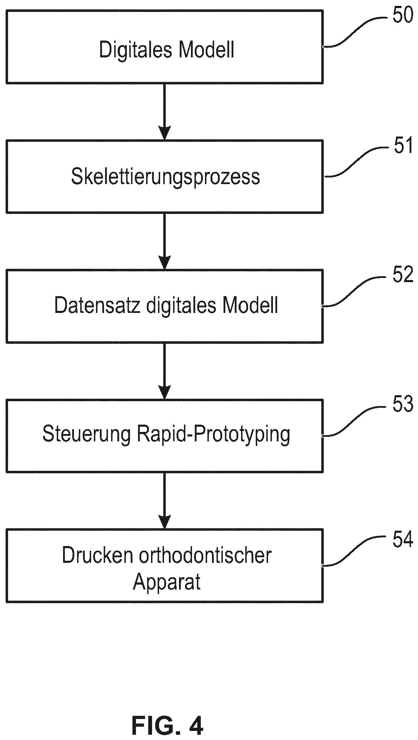

Sofern beispielsweise ein Aligner hergestellt wird, endet das erfindungsgemäße Verfahren mit dem Schritt 54. Sofern weitere Aligner herzustellen sind, wiederholen sich die Schritte 50 bis 54 gemäß Fig. 4 mit jedem einzelnen herzustellenden Aligner.

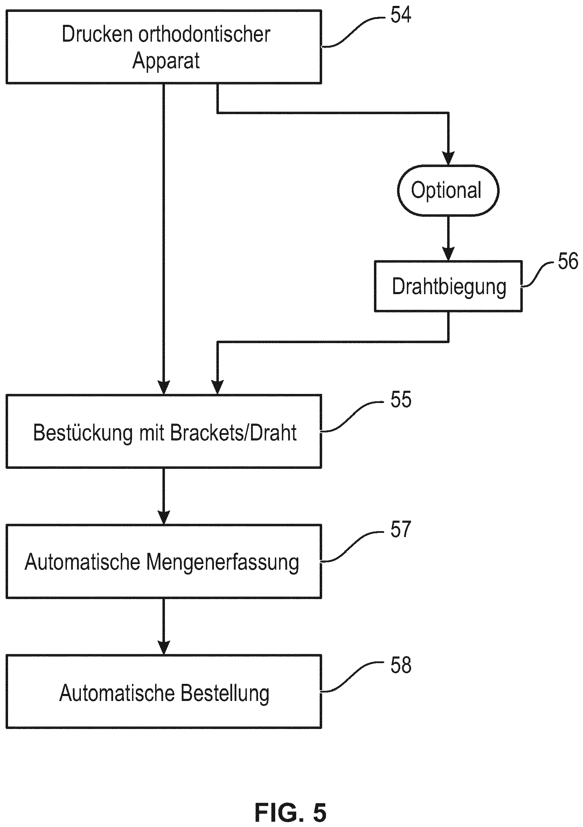

Gemäß Fig. 5 wird ein erweitertes Ausführungsbeispiel des erfindungsgemäßen Verfahrens dargestellt, sofern eine Vorrichtung zur indirekten Klebung, also ein Tray, herzustellen ist. Nach dem Drucken 54 der Kunststoffbestandteile des orthodontischen Apparates erfolgt eine automatische und/oder manuelle Bestückung 55 des gedruckten orthodontischen Apparates mit mindestens einem Bracket und/oder mindestens einem gebogenen Draht. Die Bestückung kann mit Hilfe einer im Rapid-Prototyping-Drucker befindlichen Robotik mit Greifarmen durchgeführt werden. Hierzu weist der Rapid-Prototyping-Drucker mehrere Kammern mit darin befindlichen Brackets und/oder Drähten und/oder anderen Hilfsmitteln auf. Vorzugsweise sind mehrere Brackets unterschiedlicher Ausführungsformen und/oder verschiedener Größen und/oder von unterschiedlichen Herstellern in einzelnen Kammern bevorratet. Sofern ein Draht zusammen mit den Brackets auf dem orthodontischen Apparat aufzubringen ist, muss der optionale Verfahrensschritt 56, nämlich die Biegung eines Drahtes, durchgeführt werden. Hierzu umfasst der Rapid-Prototyping-Drucker wiederum eine entsprechende Drahtbiegevorrichtung. Die Abmaße der Drähte sowie die Drahtreihenfolgen können enstprechend berechnet und abgespeichert werden. Auch eine individuelle Kennzeichnung der noch fälligen Arbeiten ist möglich, wobei in diesem Zusammenhang eine Zuordnung zu Patientendaten, welche bspw. in digitalen Patientenakten hinterlegt sind, durchgeführt werden kann.

Es sei darauf hingewiesen, dass die Bestückung auch manuell durchgeführt werden kann, d.h. der Zahnarzt bzw. Zahntechniker kann einzelne oder alle Brackets auf den dafür vorgesehenen Positionen des orthodontischen Apparates auf- oder anbringen.

In einer weiteren Ausführungsform der Erfindung kann der Rapid-Prototyping-Drucker mit einem automatischen Warenwirtschaftssystem in Verbindung stehen, sodass eine automatische Mengenerfassung von in Kammern eines Rapid-Prototyping-Druckers befindlichen Brackets und/oder Drahtes und/oder Hilfsmittel wie bspw. Schrauben und/oder verbrauchten Kunststoffmaterials erfolgen kann, so dass eine Weiterleitung der erfassten Daten an ein Warenwirtschaftssystem möglich ist (Schritt 57). Die Erkennung der einzelnen Elemente erfolgt beispielsweise mit Hilfe von RFID-Etiketten und/oder QR-Codes.

Im Rahmen des automatisierten Warenwirtschaftssystems ist es möglich automatisierte Bestellvorgänge hinsichtlich benötigter Brackets und/oder benötigten Drahtes und/oder benötigten Kunststoffmaterials zu erstellen.

Entsprechende Statistiken über die Auslastung und Produktivität des Rapid-Prototyping-Druckers kann der Benutzer jederzeit auslesen. Gleichzeitig lassen sich über die verbrauchten Materialien Rückschlüsse auf die weiteren noch notwendigen Materialien, die zur Behandlung benötigt werden, erfassen, so dass Bestellungen entsprechend angepasst werden können. Durch eine weitere Schnittstelle an die diverse Abrechnungsprogramme können dem Bediener entsprechende Abrechnungspositionen und Texte vorgeschlagen werden, so dass bei einer entsprechend vorzunehmenden Abrechnung bezüglich des hergestellten orthodontischen Apparates weitere Fehlerquellen minimiert werden können.

Es sei darauf hingewiesen, dass vorliegendes Verfahren, vorliegende Vorrichtung sowie die Anordnung sowohl zur Herstellung von Trays zur Anbringung der Brackets auf der Außenseite der Zähne als auch zur Herstellung von Trays zur Anbringung von Brackets auf der Innenseite der Zähne geeignet ist.

Die Vorrichtung zum Herstellen aller orthodontischen Apparate, insbesondere ein Rapid-Prototyping-Drucker, kann sowohl in einer Praxis als auch in einem Labor befindlich sein, wobei die Daten zur Herstellung der orthodontischen Apparate auch per Fernzugriff bzw. über eine Internetverbindung an die Rapid-Prototyping-Maschine weitergeleitet werden kann. Die fertiggestellten orthodontischen Apparate können bereits im Drucker durch eine entsprechende Verpackungseinheit verpackt werden, so dass ein hygienischer Versand bzw. eine hygienische Aufbewahrung der orthodontischen Apparate ermöglicht wird.

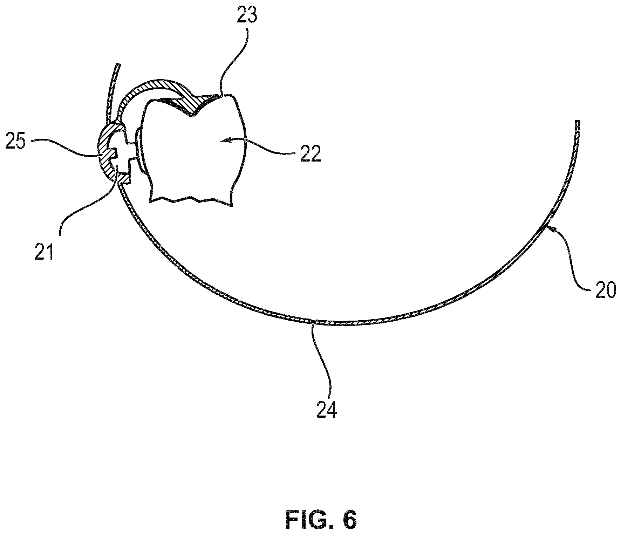

In Fig. 6 wird eine Vorrichtung zur indirekten Klebung von orthodontischen Apparaten gezeigt. Es wird ein sogenanntes Tray 20 dargestellt, welches aus Kunststoffbestandteilen besteht. An einem Zahn 22 soll beispielsweise ein Bracket 21 angebracht werden, sodass das Tray 20 an dieser, dem Zahn 22 zugewiesenen Position ein Bracket 21 aufweist. Beispielsweise kann an der zum Zahn 22 zugewandten Fläche des Brackets ein Klebstoff befindlich sein, um eine Verbindung des Brackets mit dem Zahn zu erwirken. Im Bereich des Brackets 21, ist der Kunststoff des Trays mit einer verstärkten Materialdicke ausgebildet, um einen ausreichenden Halt des Brackets 21 im Tray 20 herzustellen. Dieser verstärkte Bereich kann auch als Kappe 25 bezeichnet werden.

Des Weiteren kann das Kunststoffmaterial in Bereichen der okklusalen Zahnfläche 23 und der Schneidekante vorgesehen sein. Hierdurch wird u. a. eine exakte Positionierung des Trays 20 bzw. des Brackets 21 am Zahn 22 hergestellt. Okklusal bedeutet zur Kaufläche hin gelegen bzw. kauflächenwärts. Okklusal bedeutet auch den Kontakt zwischen Oberkieferzähnen und Unterkieferzähnen.

Außerdem kann das Kunststoffmaterial des Trays mit einer Sollbruchstelle 24 versehen sein, sodass nach dem Ankleben bzw. Anbringen der Brackets an die Zähne die Entfernung der Kunststoffmaterialien vereinfacht werden kann. Die Sollbruchstelle 24 bewirkt, dass das Tray 20 in mehreren Einzelteilen aus der Mundhöhle des Patienten 1 entnommen werden kann. Abschließend sei darauf hingewiesen, dass auch andere Materialen als Kunststoff in der 3D-Drucker-Einheit hergestellt werden können. Hierbei kann es sich beispielsweise um Keramiken oder Legierungen handeln.

### Bezugszeichenliste

- 1: Patient
- 2: intraoraler Scanner
- 3: Datenverarbeitungseinheit
- 4: Leitung
- 5: Rapid-Prototyping-Drucker
- 6: Bildschirm
- 7: Tastatur
- 8: Maus
- 9: Planungseinheit
- 10: Abdeckung
- 11: Halterung
- 12: Greifarm
- 13: Vorratsbehälter
- 14: Aufbewahrungskammer
- 20: Tray
- 21: Bracket
- 22: Zahn
- 23: okklusale Zahnfläche
- 24: Sollbruchstelle
- 25: Kappe

- 40-58: Verfahrensschritte

## Patentansprüche

1. Verfahren zur Herstellung orthodontischer Apparate, wie eine Vorrichtung zur indirekten Klebung von orthodontischen Apparaten, insbesondere Trays, und/oder Aligner und/oder herausnehmbare Zahnspangen, wobei folgende Verfahrensschritte durchgeführt werden:
- Erfassen eines orthodontisch zu behandelnden Zahnbereichs (40),
- Bereitstellen eines digitalen dreidimensionalen Modells eines orthodontisch zu behandelnden Zahnbereichs (41),
- automatische und/oder manuelle Erstellung einer modifizierten Zahnanordnung (42),
- Vergleich des Istzustands des zu behandelnden Zahnbereichs mit der zu erreichenden Endstellung der zu behandelnden Zähne,
- Berechnung, welche einzelnen Behandlungsschritte notwendig sind, um die Endposition zu erreichen,
- Unterteilung der gesamten orthodontischen Behandlung in Einzelschritte bzw. einzelne Zwischenergebnisse,
- Bereitstellung eines Datensatzes hinsichtlich der einzeln, nacheinander durchzuführenden orthodontischen Behandlungsschritte (43),
- Verknüpfung jedes einzeln, nacheinander durchzuführenden orthodontischen Behandlungsschrittes oder Zwischenergebnisses mit einem orthodontischen Apparat aus einer Auswahl von mehreren orthodontischen Apparaten (44),
- automatische und/oder manuelle Erstellung eines digitalen Modells des jeweiligen orthodontischen Apparates (50),
- Bereitstellung eines digitalen Datensatzes eines digitalen Modells des jeweiligen orthodontischen Apparates (52),
- Speicherung der digitalen Abbilder,
- Steuerung (53) eines 3D-Druckers aufgrund der digitalen Datensätze und Drucken der Kunststoffbestandteile der orthodontischen Apparate (54) und/oder von Legierungen und/oder von Keramiken,
- automatische Bestückung mindestens eines gedruckten orthodontischen Apparates mit mindestens einem Bracket und/oder mindestens einem gebogenen Draht (55) und/oder Schrauben und/oder anderen Elementen.

2. Verfahren nach Anspruch 1,
**gekennzeichnet durch** einen Skelettierungsprozess, aufgrund dessen die ausgewählten orthodontischen Apparate bezüglich überschüssigen Materials auf ein Mindestmaß digital verkleinert werden (51).

3. Verfahren nach einem der vorangegangenen Ansprüche,
**gekennzeichnet durch**
automatische Mengenerfassung (57) von in Kammern eines 3D-Druckers befindlichen Brackets und/oder Drahtes und/oder Kunststoffes und/oder Hilfselementes und Weiterleitung der erfassten Daten an ein Warenwirtschaftssystem.

4. Verfahren nach Anspruch 3,
**gekennzeichnet durch**
automatisierte Bestellvorgänge (58) hinsichtlich benötigter Brackets und/oder benötigten Drahts und/oder benötigten Kunststoffes und/oder benötigter Hilfselemente.

5. Vorrichtung zum Herstellen orthodontischer Apparate, wie eine Vorrichtung zur indirekten Klebung von orthodontischen Apparaten, insbesondere Trays, und/oder Aligner und/oder herausnehmbare Zahnspangen, welche zur Durchführung eines Verfahrens nach einem der Ansprüche 1 bis 4 ausgebildet ist, wobei die Vorrichtung mindestens eine 3D-Drucker-Einheit, einen Scanner zur digitalen Erfassung eines orthodontisch zu behandelnden Zahnbereichs und mindestens eine Datenverarbeitungseinheit (3) umfasst, wobei die Datenverarbeitungseinheit zur Steuerung der mindestens einen 3D-Drucker-Einheit ausgebildet ist und wobei die Datenverarbeitungseinheit mit dem Scanner verbunden oder verbindbar ist, wobei die mindestens eine 3D-Drucker-Einheit eine Robotik mit mindestens einer Greifvorrichtung und mindestens eine Kammer zur Befüllung mit Brackets und/oder mit Draht und/oder mit Schrauben und/oder mit anderen Elementen umfasst.

6. Vorrichtung nach Anspruch 5,
**gekennzeichnet durch**
ein automatisiertes Warenwirtschaftssystem mit einer Überwachungsvorrichtung zur Überprüfung der Füllstände der mindestens einen mit Brackets befüllten Kammer und/oder der mindestens einen mit Draht befüllten Kammer und/oder der mindestens einen mit Hilfsmitteln befüllten Kammer und/oder des zu verarbeitenden Kunststoffes.

7. Vorrichtung nach einem der Ansprüche 5 bis 6,
**dadurch gekennzeichnet, dass**
die 3D-Drucker-Einheit mindestens eine Drahtbiegevorrichtung umfasst.

8. Vorrichtung nach einem der Ansprüche 5 bis 7,
**dadurch gekennzeichnet, dass**
die 3D-Drucker-Einheit eine UV-Licht abweisende Abdeckung aufweist.

9. Vorrichtung nach einem der Ansprüche 5 bis 8,
**dadurch gekennzeichnet, dass**
die 3D-Drucker-Einheit eine Verpackungsvorrichtung aufweist.

## Claims

1. A method for producing orthodontic appliances, such as a device for the indirect sticking of orthodontic appliances, especially trays, and/or aligners and/or removable braces, wherein the following method steps are carried out:
- capturing of a tooth region to be treated orthodontically (40),
- preparation of a digital three-dimensional model of a tooth region (41) to be treated orthodontically,
- automatic and/or manual creation of a modified tooth arrangement (42),
- comparison of the actual state of the tooth region to be treated with the end position of the teeth to be treated which is to be achieved,
- calculation of which individual treatment steps are necessary in order to reach the end position,
- subdividing the entire orthodontic treatment into individual steps or individual intermediate results,
- preparation of a dataset with regard to the orthodontic treatment steps (43) which are to be carried out individually in succession,
- linkage of each orthodontic treatment step or intermediate result which is to be carried out individually in succession with an orthodontic appliance from a selection of a plurality of orthodontic appliances (44),
- automatic and/or manual creation of a digital model of the respective orthodontic appliance (50),
- preparation of a digital dataset of a digital model of the respective orthodontic appliance (52),
- storage of the digital images,
- control (53) of a 3D printer on the basis of the digital datasets and printing of the plastics material constituents of the orthodontic appliances (54) and/or of alloys and/or of ceramics,
- automatic fitting of at least one printed orthodontic appliance with at least one bracket and/or at least one bent wire (55) and/or screws and/or other elements.

2. A method according to Claim 1,
**characterised by**
a skeletonization process, on the basis of which the selected orthodontic appliances are digitally reduced to a minimum (51) with regard to excess material.

3. A method according to one of the preceding claims,
**characterised by**
automatic capturing (57) of the quantity of brackets and/or wires and/or plastics materials and/or auxiliary elements present in chambers of a 3D printer, and transmission of the captured data to an inventory control system.

4. A method according to Claim 3,
**characterised by**
automated ordering operations (58) with respect to required brackets and/or required wires and/or required plastics materials and/or required auxiliary elements.

5. A device for producing orthodontic appliances, such as a device for the indirect sticking of orthodontic appliances, especially trays, and/or aligners and/or removable braces, which is designed to carry out a method according to one of Claims 1 to 4,
wherein the device comprises at least one 3D printer unit, a scanner for digitally capturing a tooth region to be treated orthodontically and at least one data processing unit (3), the data processing unit being designed to control the at least one 3D printer unit and the data processing unit being connected or connectable to the scanner, wherein
the at least one 3D printer unit comprises a robotic system having at least one gripping device and at least one chamber for filling with brackets and/or with wire and/or with screws and/or with other elements.

6. A device according to Claim 5,
**characterised by**
an automated inventory control system having a monitoring device for checking the filling level of the at least one chamber filled with brackets and/or of the at least one chamber filled with wire and/or of the at least one chamber filled with aids and/or of the plastics material to be processed.

7. A device according to one of Claims 5 to 6,
**characterised in that**
the 3D printer unit comprises at least one wire bending device.

8. A device according to one of Claims 5 to 7,
**characterised in that**
the 3D printer unit has a UV-light resistant cover.

9. A device according to one of Claims 5 to 8,
**characterised in that**
the 3D printer unit has a packaging device.

## Revendications

1. Procédé de fabrication d'appareils orthodontiques, tel qu'un dispositif pour le collage indirect d'appareils orthodontiques, en particulier de plateaux et/ou d'alignements et/ou d'appareils de correction dentaire amovibles, dans lequel on effectue les étapes de procédé suivantes consistant à :
- détecter une zone dentaire (40) à traiter orthodontiquement,
- fournir un modèle numérique tridimensionnel d'une zone dentaire (41) à traiter orthodontiquement,
- créer automatiquement et/ou manuellement un arrangement de dents modifié (42),
- comparer l'état réel de la zone dentaire à traiter à la position finale à atteindre pour les dents à traiter,
- calculer les différentes étapes de traitement individuelles nécessaires pour atteindre la position finale,
- subdiviser l'ensemble du traitement orthodontique en étapes individuelles ou en résultats intermédiaires individuels,
- fournir un ensemble de données concernant les différentes étapes (43) du traitement orthodontique à effectuer individuellement l'une après l'autre,
- lier chaque étape individuelle du traitement orthodontique à réaliser individuellement l'une après l'autre ou chaque résultat intermédiaire avec un appareil orthodontique à partir d'une sélection de plusieurs appareils orthodontiques (44),
- créer automatiquement et/ou manuellement un modèle numérique de l'appareil orthodontique respectif (50),
- fournir un ensemble de données numériques d'un modèle numérique de l'appareil orthodontique respectif (52),
- mémoriser les images numériques,
- commander (53) une imprimante 3D en se basant sur les ensembles de données numériques et imprimer des composants en matière plastique des appareils orthodontiques (54) et/ou des alliages et/ou des céramiques,
- équiper automatiquement au moins un appareil orthodontique imprimé avec au moins un support et/ou au moins un fil incurvé (55) et/ou avec des vis et/ou avec d'autres éléments.

2. Procédé selon la revendication 1,
**caractérisé par**
un processus de squelettisation sur la base duquel les appareils orthodontiques sélectionnés sont réduits numériquement à un minimum en ce qui concerne l'excès de matériau (51).

3. Procédé selon l'une des revendications précédentes,
**caractérisé par**
un enregistrement automatique les quantités (57) de supports et/ou de fils et/ou de la matière plastique et/ou de moyens auxiliaires situés dans des chambres d'une imprimante 3D, et par une transmission des données enregistrées à un système de gestion de matières.

4. Procédé selon la revendication 3,
**caractérisé par**
des opérations de commande automatisées (58) quant aux supports nécessaires et/ou aux fils nécessaires et/ou à la matière plastique nécessaire et/ou aux éléments auxiliaires nécessaires.

5. Dispositif pour la fabrication d'appareils orthodontiques, tel qu'un dispositif pour le collage indirect d'appareils orthodontiques, en particulier de plateaux et/ou d'alignements et/ou d'appareils de correction dentaire amovibles, qui est réalisé pour mettre en oeuvre un procédé selon l'une des revendications 1 à 4,
dans lequel
le dispositif comprend au moins une unité d'imprimante 3D, un scanneur pour la détection numérique d'une zone dentaire à traiter orthodontiquement et au moins une unité de traitement de données (3), l'unité de traitement de données est réalisée pour commander ladite au moins une unité d'imprimante 3D, et
l'unité de traitement de données est connectée ou susceptible d'être connectée au scanneur,
ladite au moins une unité d'imprimante 3D comprend un système robotisé avec au moins un dispositif de préhension et au moins une chambre destinée à être remplie de supports et/ou de fil et/ou de vis et/ou d'autres éléments.

6. Dispositif selon la revendication 5,
**caractérisé par**
un système automatisé de gestion des matières, comportant un dispositif de surveillance pour contrôler les niveaux de remplissage de ladite au moins une chambre remplie de supports et/ou de ladite au moins une chambre remplie de fil et/ou de ladite au moins une chambre remplie d'éléments auxiliaires et/ou de la matière plastique à oeuvrer.

7. Dispositif selon l'une des revendications 5 à 6,
**caractérisé en ce que**
l'unité d'imprimante 3D comprend au moins un dispositif de cintrage de fil.

8. Dispositif selon l'une des revendications 5 à 7,
**caractérisé en ce que**
l'unité d'imprimante 3D comprend un recouvrement rejetant la lumière UV.

9. Dispositif selon l'une des revendications 5 à 8,
**caractérisé en ce que**
l'unité d'imprimante 3D comprend un dispositif d'emballage.
